# EUROPEAN PATENT SPECIFICATION

(11) **EP 0 874 583 B1**
(45) Date of publication and mention of the grant of the patent: **14.04.2004**
(21) Application number: 96924679.2
(22) Date of filing: 23.07.1996
(51) Int. Cl.: A61B 5/0402

(54) **DISPOSABLE ELECTRO-DERMAL DEVICE**
ELEKTRODERMALE WEGWERFVORRICHTUNG
DISPOSITIF ELECTRODERMAL JETABLE

(30) Priority: 28.07.1995 US 508928
(43) Date of publication of application: 04.11.1998
(73) Proprietor: Cardiotronics International, Inc., Orinda, California 94563 (US)
(72) Inventor: KELLY, Robert, J., Beverly Hills, CA 90210 (US); LAVINE, Thomas, Camarillo, CA 93010-8441 (US)
(74) Representative: Bauer, Wulf, Dr.
(86) International application number: PCT/US1996/012100
(87) International publication number: WO 1997/004703

(56) References cited:
- EP-A- 0 275 811
- US-A- 4 583 549
- US-A- 4 763 660
- US-A- 5 522 211

## Description

### Field Of The Invention

This invention relates to a disposable medical device employing electrical signals to monitor or stimulate various parts of the body. More particularly the present invention involves a device for establishing electrical connection to a patient's skin containing a fixed array of conductive paths of substantially the same electrical resistance for use with an electrocardiological measuring apparatus.

### Description Of The Prior Art

US 4,583,549 A (Manoli) discloses in a first embodiment shown in Fig. 1-4 a disposable electro-dermal connector device comprising: a flexible non-conductive sheet comprising a fixed array of electrical conductor strips affixed thereon and positioned in a specific size configuration normally used for standard electrocardial recording. The conductor strips have receptor ends V1 to V6 adapted for electrical connection with the skin for receiving and transmitting electrical impulses and a terminal connection end which is adapted for connection with an electrocardiological measuring apparatus. The receptor pads V1 and V2 are attachable approximately on either side of the sternum at the fourth intercostal space. Receptor pad V3 is attachable midway between V2 and V4. Not in this embodiment, but in the discussion of the state of the art Manoli states to arrange V5 equidistant between V4 and V6.

U.S. Patent No. 4,328,814 to Arkam teach a plurality of electrodes attached to a single junction connector having one cable leading to the EKG device. This device is designed for an adult patient so that patients having larger or smaller torsos will have difficulty in using the device because the electrodes cannot be easily adjusted to accommodate a smaller or larger torso. Also, in the event of a heart attack, the plurality of electrodes must be disconnected from the EKG device by disconnecting the main connectors and then detaching the plurality of the electrodes. No electrodes remain on the patient to monitor the heart attack.

U.S. Patent No. 4,353,372 to Ager discloses a plurality of electrodes which plug into a junction box connected to an EKG machine. Each of the electrodes includes wires molded into a central cable system which joins the junction box. This device, does not include means for quickly attaching or removing the electrodes. For example, in an emergency situation if the electrodes must be removed quickly, the junction box must be disconnected first and then each of the electrodes must be detached. Although each electrode has a wire lead from the main molded cable, which may permit some adjustment in the placement of the electrodes on the upper portion of a human torso, the device is not entirely adequate for large adults or very small children because of the limited adjustment of each electrode.

U.S. Patent No. 4,608,987 to Mills relates to a vest-like garment having a plurality of apertures adapted for receiving associated electrodes. However, the vest is not tailored for a specific patient and proper fit is provided by adjustable straps which may be secured by VELCRO^{R} material. Therefore, there is no assurance that the electrodes are placed at the same anatomical location upon reuse with the same patient.

U.S. Patent No. 3,910,260 describes telephonic units for transmitting ECG signals to ECG receiving equipment which could be at a hospital or a physician's office. The transmission may take place in emergency vehicles where prior medical history may not be readily available. In order to obtain meaningful and reliable data ECG signals are necessary for the care providers. None of the prior art devices have disclosed a low cost solution for obtaining repeatable placement of sensors for accurate and readable ECG signals in the field by unskilled individuals.

Because of the inadequacies of prior art devices there is a need for a system which prevents EKG electrode leads from being entangled; provides quick removal of some of the electrodes while leaving the remaining electrodes in position when it is necessary to administer aid to a patient having a heart attack; provides accurate repeatable placement of electrodes at substantially the same anatomical location; accurately and repeatedly obtains signals from body electrodes by efficient and effective electrical transmission; may be attached by unskilled persons; and may be available in various sizes to accommodate to fit the patient.

### Summary Of The Invention

The present invention, in the broadest sense involves a disposable non-conducting flexible sheet incorporating a fixed array of electrical conducting strips emanating from a terminus that can connect to a standard electrocardiographic cable or telemetric unit. The strips are used as both collectors and as transmitters for electrical impulses. Conventional sensory electrodes are optional since the device can function without them. More particularly, the invention relates to a disposable, electro-dermal connector device comprising: a flexible non-conductive sheet comprising a fixed array of electrical conductor strips affixed thereto having a receptor pad end and a terminal connection end said array positioned in the configuration normally used for electrocardial recording whereby the flexibility of said connector and adhesion of the surface of the flexible sheet to skin are substantially enhanced.

Each strip includes a first end portion or receptor end adapted for electrical connection with the skin for receiving electrical impulses. A second end portion terminates in a common electrical connection or cable junction which is adapted for connection with a standard type of cable junction for connection with the electrocardiograph device.

The conductive strips may be printed on the single layer non-conductive film or sheet by any conventional printing or silk screening type of process. The portion of the strip which need not be exposed can be coated or covered with a non-conductive coating or adhesive material which can be cured. Conductor strips which are less than 10 micrometers in thickness which provide enhanced flexibility without distorting the electrical signal.

More particularly, the invention relates to a disposable electro-dermal connector device comprising: a flexible non-conductive sheet comprising a fixed array of electrical conductor strips affixed thereon and positioned in a specific size configuration normally used for standard electrocardial recording, said conductor strips having a receptor pad end adapted for electrical connection with the skin for receiving electrical impulses and a terminal connection end which is adapted for connection with an electrocardiological measuring apparatus, wherein receptor pads V₁ and V₂ are attached approximately on either side of the sternum at the fourth intercostal space and receptor pad V₃ is attached over the fifth intercostal space midway between V₂ and V₄. V₅ is equidistant between V₄ and V₆. The distance between V₁ and V₂, V₂ and V₃ and V₄ is about 4,45 ± 1,42 cm (1.75 inches ± 0.56 inch).

### Brief Description Of The Drawings

FIG. 1 shows a preferred device of the present invention for attachment to the torso of a patient.
FIG. 2 shows a preferred embodiment of the present invention of properly positioning the device on a patient.
FIG. 3 illustrates the first step in the method for determining the size of the device to be used on a patient according to this invention.
FIG. 4 shows the second step in the method for determining the size of the device to be placed on a patient according to this invention.

### Description Of The Preferred Embodiments

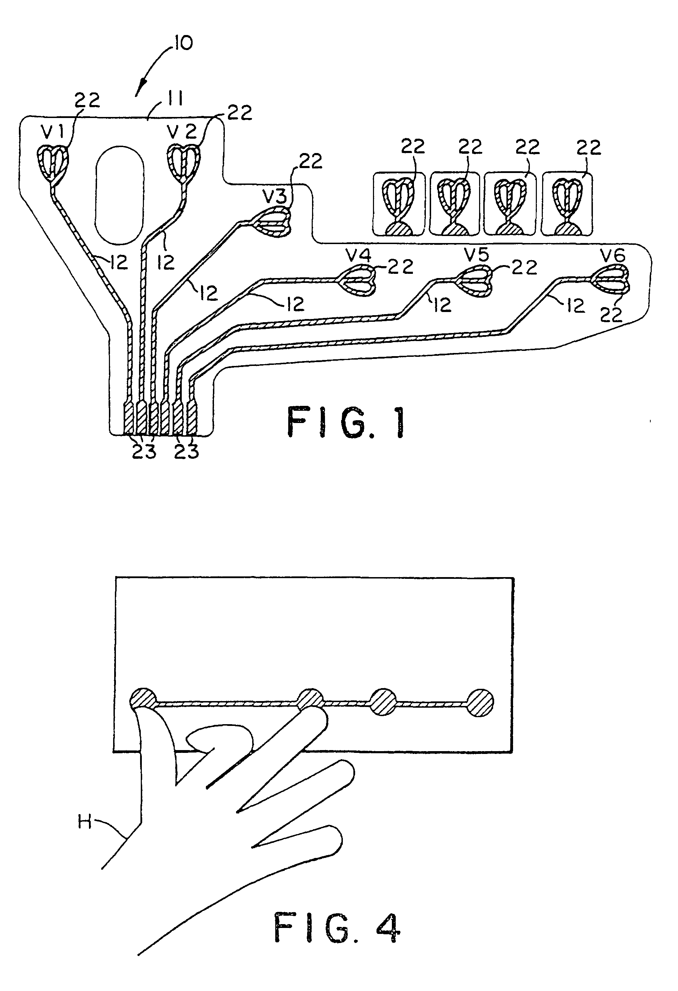

Referring now to the drawings, FIG. 1 illustrates the electro-dermal connector device **10** of the present invention for placement on the chest of a patient comprising flexible non-conducting sheet **11** incorporating multiple conductor strips **12** for connection to a standard electrocardiographic receiving unit. The non-conducting sheet **11** includes conductor strips **12** which form end sensors or receptor **22** which are positioned on the sheet and spaced relative to each other whereby each receptors **22** is positioned in a specific size configuration normally used for electrocardial recordings.

Each strip **12** includes a first end portion or receptor **22** adapted for electrical connections with the skin for receiving and transmitting electrical impulses generated by the body. A second end of each strip **12** or the terminal connector end **23** to engage a common electrical connection or cable junction (not shown) for connection with the electrocardiograph device (not shown).

When in use an electrically conductive ion containing a biocompatible adhesive gel is applied to the body contacting side of sheet **11** at each receptor **22** of connector **12** for adhesion to the skin of the patient for providing electrical connection between each of the precordial ends and the terminal end **23** connected to the proper receiving devices (not shown).

The adhesive gel coated area of connector device includes at least one release liner in releasable adhesive contact with the gel. Each of the conductor strips **12** are less than **10**, preferably less than 5 micrometers in thickness whereby the flexibility of the connector and adhesion of the gel surface to the skin are substantially enhanced.

FIG. 1 shows the connector array (V₁, V₂, V₃, V₄, V₅, and V₆) on flexible sheet **11** which is designed to adhere to a human torso so that the terminal ends **23** are located below the sternal notch, over the ribs and at the side of the torso. The flexible sheet **11** can be substantially transparent and includes an opening in the proximate center which is intended to span the upper portion of the sternum of the patient. The sheet may include indicia adjacent to or on each of the conductor strips to facilitate correct placement of the receptors on the precordial areas of the human torso.

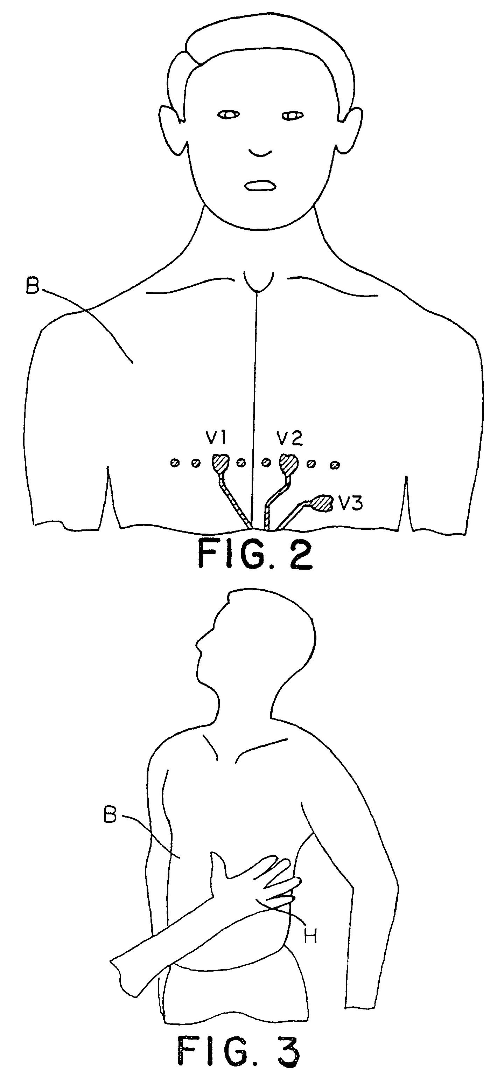

FIG. 2 illustrates the position of the electro-dermal connector device **10** as it is properly positioned upon a patient. The connector device **10** is generally attached by adhering the precordial receptors. The receptors V₁ and V₂ are attached approximately on opposite sides of the sternum at the fourth intercostal space. Pads V₃ and V₄ are attached over the ribs. Pads V₅ and V₆ are placed at the side of the torso so that V₅ is midway between V₄ and V₆. For small sizes the distance between V₄ and V₆ is on the average 8,89 cm (3.5 inches), for medium 12,7 cm (5.0 inches) and for large 17,8 cm (7 inches). The contour of the electro-dermal connector **10** is configured to conform substantially to the shape of a human trunk.

In cross section a preferred laminate of the invention comprises the following layers:
a) a flexible non-conductive film of polyethylene terphthalate;
b) a catalyst layer in contact with silver ink;
c) a connector strip in contact with silver ink;
d) a dielectric layer in contact with silver ink and silver chloride receptor layer superimposed upon the silver ink layer;
e) a conductive hydrogel layer superimposed upon the silver chloride receptor layer; and
f) a flexible release liner as the top layer superimposed upon the conductive hydrogel.

The flexible non-conductive web or sheet **11** may be formed from any non-conductive flexible natural or synthetic sheet material which is capable of accepting a print. Generally any cellulosic material, polyester, polyolefin, polyvinyl chloride, nylon or mixtures thereof would be suitable. Preferably, cotton, polypropylene, polyethylene can be used because of cost. Polyethylene terphthalate is most preferred. The polymer sheet material may be color coded for specific body areas or may contain an outline and/or color markings to simplify the electro-dermal connector device. As mentioned earlier the device of this invention is designed to include the use by an untrained or trained individual. This device allows an untrained person including the patients themselves to provide highly reliable and repeatable ECG signals.

The receptors **12** can be produced from any electrically conductive material, e.g., metals, conductive polymers, graphite, carbon fibers and the like. Conductive materials such as gold, copper, silver, tin, aluminum, N-vinyl pyrrolidone and alloys or mixtures thereof maybe used. The receptors can be made of metal foil or made from a conductive paste of a metal in particle form in a suitable binder which is printed or silk screened onto the flexibly non-conductive sheet. The connective polymer may be heat pressed or otherwise conventionally adhered to the web or sheet.

Preferably, copper strips are electrolessly deposited on the polymeric sheets in a range from about 0.25 to about 5 microns, more preferably from 0.25 to 1.5 microns and most preferably 0.4 microns in thickness.

If desired, the exposed conductive strips may be partially coated with a dielectric polymeric material so that only selective portions are exposed, Suitable dielectric coatings include polyesters, ethylene-vinyl acetate copolymers, polyvinyl chloride and its copolymers, terpolymers such as acrylonitrile-butadiene styrene (ABS resins) and inter alia.

One form of metallic ink which may be used is a silver ink is commercially available and marketed by Dupont Chemical Corp. of Wilmington, Delaware under the tradename Composition 9793.

The conductive adhesive hydrogel is sold commercially by Lee Tec Corporation of Eden Prairie, MN. Other suitable conductive adhesives are manufactured by 3M Corporation of St. Paul, MN. Although an adhesive hydrogel is preferred any commercial electro-dermal adhesive would be operable. Preferably the area size of the hydrogel is between about 3 and 9 square centimeters.

The flexible release liner may be made from a suitable dielectric film which includes polyesters, olefinic polymers, polyvinyl chloride and its copolymers, acrylic rubbers, ABS resin and the like.

In a preferred embodiment the electro-dermal connector device 10 comprises at least six gel contact areas and is adapted for use in electrocardiography.

The electro-dermal connector device **10** is available in sizes to accommodate any size adult person. It has been found that the distance between pads V₁ to V₄ is constant for all sizes. The separation of 4,45 cm (1.75 inches) will accommodate all adults with a tolerance of plus or minus 1,42 cm (0.56 inch) at each pad. It has also been found that body placement for pads V₅ and V₆ vary depending on individual size. FIG. 4 shows a method of determining the proper size. The measurement from the V₄ position to V₆ position determines the size of the device. As illustrated in FIG. 4 this measurement is the distance determined between the thumb and the middle finger and then matched to a scale provided. The table below corresponds to the illustrated scale.

**TABLE**

| SIZE | V₄ - V₅ | V₅ - V₆ |
|---|---|---|
| Small | 4,45 cm | 4,45 cm |
| Medium | 6,35 cm | 6,35 cm |
| Large | 8,89 cm | 8,89 cm |

Generally, the distance between V₄ to V₆ will be determined by the size of the patient, that is, the size of the vest. For a small vest the distance between V₄ and V₆ is about 6,35 to 11,43 cm (2.5 to 4.5 inches) with the centering of V₅ being at about 4,45 cm (1.75 inches), the medium vest has a distance of about 10,16 to 15,24 cm (4.0 to 6.0 inches) with the centering of V₆ being about 6,35 cm (2.5 inches), and the large vest the distance is about 15,24 to 20,32 cm (6.0 to 8.0 inches) with the centering being about 8,9 cm (3.5 inches).

In all sizes of the devices of the invention V₁, V₂, V₃ and V₄ are all positioned the same. The center of V₁ is located on a radius of 2,1 cm (0.825 inches) from a point 4,45 ± 1,42 cm (1.75 inches ± 0.56 inches) from the center of V₂ on the 270 (90) degree radial from the center of V₂ wherein the radial is measured with zero degrees from the top of the device. The center of V₃ is located within a radius of 2,1 cm (0.825 inch) from a point 4,45 ± 1,42 cm (1.75 inches ± 0.56 inches) from the center V₂ on the top 236 (56) degree radial from the center of V₂. The center of V₄ is located within a radius of 2,1 cm (0.825 inches) from a point 8,9 cm (3.5 inches) from the center V₂ on the 236 (56) degree radial from the center of V₂.

A typical dimensional layout for ₅ and V₆ relative to V₄ is as follows:

**Table**

| SIZE | V₄ - V₅ | V₅ - V₆ |
|---|---|---|
| Small Vest | 4,45 cm | 4,45 cm |
| Medium Vest | 6,35 cm | 6,35 cm |
| Large Vest | 8,89 cm | 8,89 cm |

The distance between V₁ and V₂ is 4,45 cm (1.75 inches), the distance between sternum and V₄ along a horizontal line is 9,78 cm (3.85 inches) with V₃ along a horizontal line being equidistant from V₂ and V₄.

## Claims

1. A disposable electro-dermal connector device (10) comprising:
a flexible non-conductive sheet (11) comprising a fixed array of electrical conductor strips (12) affixed thereon and positioned in a specific size configuration normally used for standard electrocardial recording, said conductor strips (12) having receptor ends V₁ to V₆ adapted for electrical connection with the skin for receiving and transmitting electrical impulses
and a terminal connection end (23) which is adapted for connection with an electrocardiological measuring apparatus wherein receptor pads V₁ and V₂ are attachable approximately on either side of the sternum at the fourth intercostal space **characterized in that** receptor pad V₃ is attachable over the fifth intercostal space midway between V₂ and V_{4'} the distance between V₁ and V₂, V₂ and V₃ and V₃ and V₄ is 4,45 ± 1,42 cm (1.75 inches ± 0.56 inch), and V₅ is equidistant between V₄ and V₆.

2. The connector device of claim 1 wherein the material of said sheet (11) is selected from non-conductive flexible natural or synthetic sheet material.

3. The connector device of claim 2 wherein said non-conductive sheet (11) is selected from cellulosic materials, polyesters, polyolefins, polyvinyl chloride or nylon.

4. The connector device of claim 3 where said cellulosic material is cotton or paper.

5. The connector device of claim 3 wherein said polyester is polyethylene terphthalate.

6. The connector device of claim 1 wherein the conductor strips (12) are selected from metal, polymer, graphite or carbon fibers.

7. The connector device of claim 6 wherein said metal is selected from gold, copper, silver, tin or aluminum and alloys or mixtures thereof.

8. The connector device of claim 7 wherein said conductor strips are made of metal foil or a metal paste.

9. The connector device of claim 8 wherein said paste is printed or silk screened onto said non-conductive sheet (11).

10. The connector device of claim 7 wherein said paste is electrolessly deposited on the non-conductive sheet (11).

11. The connector device of claim 6 wherein the exposed conductive strips (12) are coated with a dielectric polymeric material so that only sensitive portions are exposed.

12. The connector device of claim 11 wherein said dielectric polymeric material is selected from polyesters, copolymers of ethylene-vinyl acetate, homopolymers and copolymers of polyvinylchloride and ABS resins.

13. The connector device of claim 7 wherein said conductor strips (12) are less than 10 microns in thickness.

14. The connector device of claim 1 wherein the distance between V₄ and V₅ and V₅ and V₆ is about 4,45 cm (1.75 inches).

15. The connector device of claim 1 wherein the distance between V₄ and V₅ and V₅ and V₆ is about 6,35 cm (2.5 inches).

16. The connector device of claim 1 wherein the distance between V₄ and V₅ and V₅ and V₆ is about 8,89 cm (3.5 inches).

17. The connector device of claim 1 wherein V₁ is located 4,45 ± 1,42 cm (1.75 inches ± 0.56 inches) in radius on the 270 degree radial from the center of V₂.

## Patentansprüche

1. Elektrodermale Anschlussvorrichtung (10), die wegwerfbar ist und aufweist:
ein flexibles, nicht leitendes Blatt (11), das eine vorgegebene Anordnung elektrischer Leiterstreifen (12) hat, die auf ihm befestigt und in einer speziellen Größenkonfiguration positioniert sind, wie sie üblicherweise für gängige elektrokardiale Aufzeichnungen verwendet wird, diese Leiterstreifen (12) haben Rezeptorenden in V₁ bis V₆, die für eine elektrische Verbindung mit der Haut ausgelegt sind für Empfang und Senden elektrischer Impulse und ein abschließendes Verbinderende (23), das für eine Verbindung mit einem elektrokardiologischen Messapparat ausgelegt ist, wobei Rezeptorflächen V₁ und V₂ angebracht werden können ungefähr auf jeder Seite des Sternums am vierten Zwischenrippenraum,
**dadurch gekennzeichnet, dass** der Rezeptorfleck V₃ über dem fünften Zwischenrippenraum anbringbar ist auf der Mitte zwischen V₂ und V₄, der Abstand zwischen V₁ und V₂, V₂ und V₃ und V₃ und V₄ 4,45 ± 1,42 cm (1,75 Zoll ± 0,56 Zoll) beträgt, und V₅ gleichabständig zwischen V₄ und V₆ ist.

2. Die Anschlussvorrichtung nach Anspruch 1, wobei das Material des Blattes (11) ausgewählt ist aus einem natürlichen oder synthetischen nicht leitenden, flexiblen Blattmaterial

3. Die Anschlussvorrichtung nach Anspruch 2, wobei das nicht leitende Blatt (11) ausgewählt ist aus Zellulosematerialien, Polyestern, Polyolefinen, Polyvinylclorid oder Nylon.

4. Die Anschlussvorrichtung nach Anspruch 3, wobei das Zellulosematerial Baumwolle oder Papier ist.

5. Die Anschlussvorrichtung nach Anspruch 3, wobei der Polyester ein Polyethylenterphthalat ist.

6. Die Anschlussvorrichtung nach Anspruch 1, wobei die Leiterstreifen (12) ausgewählt sind aus Metall, Polymer, Grafit oder Kohlenstofffasern.

7. Die Anschlussvorrichtung nach Anspruch 6, wobei das Metall ausgewählt ist aus Gold, Kupfer, Silber, Zinn oder Aluminium und Legierungen oder Mischungen dieser Metalle.

8. Die Anschlussvorrichtung nach Anspruch 7, wobei die Leiterstreifen gefertigt sind aus Metallfolie oder aus einer Metallpaste.

9. Die Anschlussvorrichtung nach Anspruch 8, wobei die Paste gedruckt oder siebgedruckt ist auf das nicht leitende Blatt (11).

10. Die Anschlussvorrichtung nach Anspruch 7, wobei die Paste stromlos auf das nicht leitende Blatt (11) aufgetragen ist.

11. Die Anschlussvorrichtung nach Anspruch 6, wobei die exponierten leitenden Streifen (12) mit einem dielektrischen Polymermaterial bedeckt sind, und dass nur die sensitiven Bereiche exponiert sind.

12. Die Anschlussvorrichtung nach Anspruch 11, wobei das dielektrische polymerische Material ausgewählt ist aus Polyestern, Copolymeren von Ethylen-Vinylacetat, Homopolymeren und Copolymeren von Polyvinylchlorid und ABS-Harzen.

13. Die Anschlussvorrichtung nach Anspruch 7, wobei die Leiterstreifen (12) eine Dicke von weniger als 10 Mikrometern aufweisen.

14. Die Anschlussvorrichtung nach Anspruch 1, wobei der Abstand zwischen V₄ und V₅ und V₅ und V₆ ungefähr 4,45 cm (1,75 Zoll) beträgt.

15. Die Anschlussvorrichtung nach Anspruch 1, wobei der Abstand zwischen V₄ und V₅ und V₅ und V₆ ungefähr 6,35 cm (2,5 Zoll) beträgt.

16. Die Anschlussvorrichtung nach Anspruch 1, wobei der Abstand zwischen V₄ und V₅ und V₅ und V₆ ungefähr 8,89 cm (3,5 Zoll) beträgt.

17. Die Anschlussvorrichtung nach Anspruch 1, wobei V₁ radial positioniert ist bei 4,45 ± 1,42 cm (1,75 Zoll ± 0,56 Zoll) zum Zentrum von V₂ über einen Bogen von 270°.

## Revendications

1. Dispositif de connexion électro-dermique (10) jetable comprenant :
une feuille non conductrice souple (11) comportant un agencement fixe de pistes conductrices d'électricité (12) assujetties à celle-ci et disposées selon un montage spécifique utilisé normalement pour des enregistrements électrocardiologiques standards, les pistes conductrices (12) ayant des extrémités réceptrices V₁ à V₆ susceptibles d'être connectées électriquement à la peau pour recevoir et transmettre des impulsions électriques ainsi qu'une borne d'extrémité (23) qui est apte à être branchée à un appareil de mesure de l'électrocardiogramme, des électrodes réceptrices V₁ et V₂ étant susceptibles d'être appliquées approximativement de chaque coté du sternum au niveau du quatrième espace intercostal, **caractérisé en ce que** l'électrode réceptrice V₃ est susceptible d'être appliquée au-dessus du cinquième espace intercostal à mi-chemin entre V₂ et V₄, l'écartement entre V₁ et V₂, V₂ et V₃ et V₃ et V₄ étant de 4,45 ± 1,42 cm (1,75 pouces ± 0,56 pouces) et V₅ étant équidistant entre V₄ et V₆.

2. Dispositif de connexion selon la revendication 1, **caractérisé en ce que** le matériau de la feuille (11) est choisi parmi les matériaux en feuille naturels ou synthétiques souples, non conducteurs.

3. Dispositif de connexion selon la revendication 2, **caractérisé en ce que** la feuille non conductrice (11) est choisie parmi les matériaux cellulosiques, les polyesters, les polyoléfines, le chlorure de polyvinyle ou le nylon.

4. Dispositif de connexion selon la revendication 3, où le matériau cellulosique est du coton ou du papier.

5. Dispositif de connexion selon la revendication 3, **caractérisé en ce que** le polyester est du polyéthylène téréphtalate.

6. Dispositif de connexion selon la revendication 1, **caractérisé en ce que** les pistes conductrices (12) sont choisies parmi le métal, le polymère, les fibres de graphite ou de carbone.

7. Dispositif de connexion selon la revendication 6, **caractérisé en ce que** le métal est choisi parmi l'or, le cuivre, l'argent, l'étain ou l'aluminium et les alliages et mélanges de ceux-ci.

8. Dispositif de connexion selon la revendication 7, **caractérisé en ce que** les pistes conductrices sont faites d'une feuille de métal ou d'une pâte métallique.

9. Dispositif de connexion selon la revendication 8, **caractérisé en ce que** la pâte est imprimée ou imprimée en sérigraphie sur la feuille non-conductrice (11).

10. Dispositif de connexion selon la revendication 7, **caractérisé en ce que** la pâte est appliquée sur la feuille non conductrice (11) par dépôt sans courant électrique.

11. Dispositif de connexion selon la revendication 6, **caractérisé en ce que** les pistes conductrices (12) exposées sont revêtues d'un matériau polymérique diélectrique de manière à n'exposer que les parties sensibles.

12. Dispositif de connexion selon la revendication 11, **caractérisé en ce que** le matériau polymérique diélectrique est choisi parmi les polyesters, les copolymères éthylène/acétate de vinyle, les homopolymères et copolymères de chlorure de polyvinyle et les résines ABS.

13. Dispositif de connexion selon la revendication 7, **caractérisé en ce que** les pistes conductrices (12) ont une épaisseur inférieure à 10 micromètres.

14. Dispositif de connexion selon la revendication 1, **caractérisé en ce que** la distance entre V₄ et V₅ et V₅ et V₆ est d'environ 4,45 cm (1,75 pouces).

15. Dispositif de connexion selon la revendication 1, **caractérisé en ce que** la distance entre V₄ et V₅ et V₅ et V₆ est d'environ 6,35 cm (2,5 pouces).

16. Dispositif de connexion selon la revendication 1, **caractérisé en ce que** la distance entre V₄ et V₅ et V₅ et V₆ est d'environ 8,89 cm (3,5 pouces).

17. Dispositif de connexion selon la revendication 1, **caractérisé en ce que** V₁ est situé radialement à 4,45 ± 1,42 cm (1,75 pouces ± 0,56 pouces) du centre de V₂, sur le rayon de 270 degrés.
